# EUROPÄISCHE PATENTSCHRIFT

(11) **EP 1 720 818 B1**
(45) Veröffentlichungstag und Bekanntmachung des Hinweises auf die Patenterteilung: **15.09.2010**
(21) Anmeldenummer: 05801747.6
(22) Anmeldetag: 27.10.2005
(51) Int. Cl.: C07C 29/149

(54) **VERFAHREN ZUR HERSTELLUNG VON HEXANDIOL - 1,6**
METHOD FOR PRODUCING 1,6 HEXANEDIOL
PROCÉDÉ POUR PRODUIRE DU 1,6-HEXANEDIOL

(30) Priorität: 05.11.2004 DE 102004054047
(43) Veröffentlichungstag der Anmeldung: 15.11.2006
(73) Patentinhaber: BASF SE, 67056 Ludwigshafen (DE)
(72) Erfinder: SIRCH, Tilman, 67105 Schifferstadt (DE); TEBBEN, Gerd-Dieter, 37073 Göttingen (DE); HECK, Ludwig E., 68535 Edingen-Neckarhausen (DE); DIEFENBACHER, Armin, 76726 Germersheim (DE); KRAUSE, Alfred, 67346 Speyer (DE); BORGEL, Franz, 67246 Dirmstein (DE)
(86) Internationale Anmeldenummer: PCT/EP2005/011489
(87) Internationale Veröffentlichungsnummer: WO 2006/048170

(56) Entgegenhaltungen:
- WO-A-97/31882

## Beschreibung

Die Erfindung betrifft ein Verfahren zur Herstellung von Hexandiol-1,6 aus einem Adipinsäure und 6-Hydroxycapronsäure enthaltenden Carbonsäuregemisch, das bei der Oxidation von Cyclohexan zu Cyclohexanon/Cyclohexanol mit Sauerstoff und durch Wasserextraktion des Reaktionsgemisches erhalten wird, durch Veresterung der Säuren mit C₁- bis C₁₀-Alkoholen und Hydrierung, wobei man aus einem nach der Veresterung und/oder nach der Hydrierung gewonnenen Gemisch aus dem Veresterungsalkohol und Leichtsiedern den Alkohol durch ein Membransystem abtrennt und in die Veresterung zurückführt.

Aus WO 97/31883 ist ein Verfahren bekannt, Hexandiol-1,6- aus wässrigen Lösungen von Carbonsäuren herzustellen, die bei der Oxidation von Cyclohexan zu Cyclohexanon/Cyclohexanol mit Sauerstoff und Wasserextraktion anfallen, wobei man
a) die in dem wässrigen Dicarbonsäuregemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag unter Abtrennung eines Alkohol-Leichtsieder-Gemischs in an sich bekannter Weise Hexandiol-1,6 gewinnt.

Die wässrigen Lösungen von Carbonsäuren, die bei der Oxidation von Cyclohexan zu Cylcohexanol und Cyclohexanon (vgl. Ullmann's Encyclopedia of Industrial Chemistry, 5. Ed., 1987, Vol. A8, S. 49) als Nebenprodukte entstehen, im folgenden Dicarbonsäurelösung (DCL) genannt, enthalten (berechnet wasserfrei in Gew.-%) im allgemeinen ziwschen 10 und 40 % Adipinsäure, zwischen 10 und 40 % 6-Hydroxycapronsäure, zwischen 1 und 10 % Glutarsäure, zwischen 1 und 10 % 5-Hydroxyvaleriansäure, zwischen 1 und 5 % 1,2-Cylcohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im allgemeinen 5 % nicht übersteigen. Beispielsweise seien Essigsäure, Propionsäure, Buttersäure, Valeriansäure, Capronsäure, Oxalsäure, Malonsäure, Bernsteinsäure, 4-Hydroxybuttersäure und γ-Butyrolacton genannt.

Um das Verfahren gemäß WO 97/31883 möglichst wirtschaftlich zu betreiben, ist es sinnvoll den Veresterungsalkohol ROH zurückzugewinnen und immer wieder in die Veresterung einzusetzen, das heißt eine niedrige Alkoholeinsatzzahl zu bewirken.

Der Veresterungsalkohol wird in zwei Stufen des Verfahrens zurückgewonnen und fällt jeweils im Gemisch mit Leichtsiedern an. In Stufe b) des Verfahrens nach der Veresterung wird ein Alkohol-Leichtsieder-Gemisch zurückgewonnen, das im allgemeinen aus etwa 80 Gew.-% Veresterungsalkohol, 10 Gew.-% Wasser und einem aus leichtsiedenden Estern und Ethern bestehenden Rest besteht. Nach der Hydrierung wird in Stufe e) ein Alkhol-Leichtsieder-Gemisch gewonnen, das zu etwa 80 Gew.-% aus dem Veresterungsalkohol und 5 Gew.-% leichtsiedenden Ethern sowie einem Rest weiterer Alkohole besteht.

Der leichtsiedende Etheranteil ist im wesentlichen aus 2-Methyltetrahydropyran (mTHP), Tetrahydropyran (THP), 2-Methyltetrahydrofuran (mTHF), 2-Ethyltetrahydrofuran (ETHF), Tetrahydrofuran (THF) und Hexamethylenoxid zusammengesetzt. Die Rückführung dieser Komponenten in die Veresterung würde zu einer Aufpegelung der Etherkomponenten im Veresterungsalkohol führen, die zu erhöhten Energieaufwand in der folgenden Kolonne und zu geringerem Alkoholüberschuss und somit zu einem schlechterem Umsatz in der Veresterung führt.

Zur Vermeidung dieser Aufpegelung der Etherkomponenten werden gemäß WO 97/31883 die Alkohol-Leichsieder-Gemische aus den Stufen b) und/oder e) einer Destillation in einer Kolonne unterzogen. Nachteilig an diesem Verfahren ist, dass die Abtrennung der Ether sich als unvollständig erweist. Es wird daher insbesondere bei kontinuierlicher Durchführung des Hexandiol-Herstellungsverfahrens trotz der Destillation erforderlich einen Teil des zurückgewonnen Alkohols (Rückalkohol) auszuschleusen und durch frischen Veresterungsalkohol zu ersetzen. Der ausgeschleuste Teil des Rückalkohols beträgt bei kontinuierlicher Fahrweise etwa 2,2 Gew.-% des der Veresterung zugeführten Rückalkohols. Der ausgeschleuste Teil des Rückalkohols wird in der Regel kostenintensiv verbrannt.

Es bestand daher die Aufgabe, ein Verfahren vorzuschlagen, dass die genannten Nachteile vermeidet.

Diese Aufgabe wurde erfindungsgemäß gelöst mit einem Verfahren zur Herstellung von Hexandiol-1,6 aus einem Adipinsäure, 6-Hydroxycapronsäure und geringe Mengen 1,4-Cyclohexandiole enthaltenden Carbonsäuregemisch, das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemischs erhalten wird, durch Veresterung der Säuren und Hydrierung, wobei man
a) die in dem wässrigen Dicarbonsäuregemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag unter Abtrennung eines Alkohol-Leichtsiedergemischs in an sich bekannter Weise Hexandiol-1,6 gewinnt, wobei aus den nach der Veresterung in Stufe a) und/oder nach der Hydrierung in Stufe e) gewonnenen Gemischen aus Alkoholen und Leichtsiedern durch ein Membransystem Alkohol abgetrennt und ganz oder teilweise , bevorzugt jedoch ganz, in die Veresterung zurückgeführt wird.

Die erfindungsgemäße Auftrennung des Alkohol-Leichtsieder-Gemischs mit dem Membransystem ermöglicht die Verhinderung der Aufpegelung der Etherfraktion bei der Rückführung des Veresterungsalkohols durch eine effektivere Abtrennung der Ether. Die Rückalkoholausschleusung zur Verbrennung des Alkohols kann im allgemeinen von etwa 2,2 Gew.-% nach dem in WO 97/31883 bekannten Destillationsverfahren auf 0,6 Gew.-% gesenkt werden. Das erfindungsgemäße Verfahren ist durch die Einsparung von Veresterungsalkohol und Verbrennungskosten deutlich wirtschaftlicher, was insbesondere für großtechnische Anlage ein erheblicher Vorteil ist.

Das erfindungsgemäße Verfahren ist abgesehen von der erfindungsgemäßen Trennung des Alkohol Leichtsieder-Gemischs mit Hilfe eines Membransystems in allen Einzelheiten in WO 97/31883 beschrieben, so dass auf diese Schrift ausdrücklich Bezug genommen wird. Sämtliche dort gemachten Angaben sollen auch hier ohne irgendwelche Beschränkungen gelten.

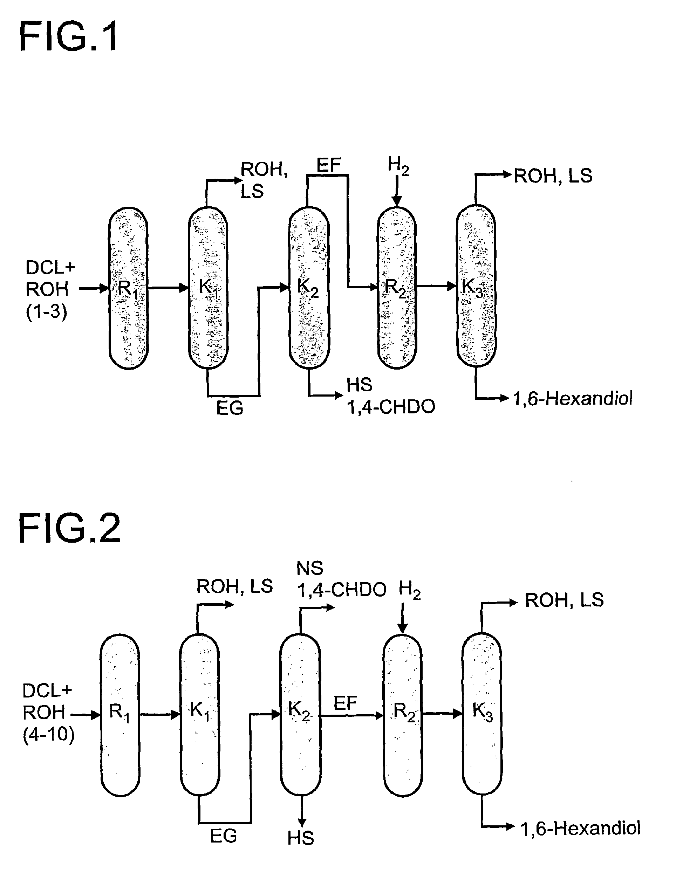

Das dort beschriebene Verfahren wird mit seinen Varianten A (Fig. 1) und Variante B (Fig. 2) hier nochmals erläutert (wobei die Begriffe über Kopf bzw. als Sumpf jeweils den Abzug oberhalb bzw. unterhalb des Zulaufs bedeuten),:

### Variante A

Wie in Fig. 1 dargestellt, wird die Dicarbonsäurelösung (DCL), gegebenenfalls nach Entwässerung, zusammen mit einem C₁- bis C₃-Alkohol, vorzugsweise Methanol, in den Veresterungsreaktor R₁ eingespeist, wo die Carbonsäuren verestert werden. Das erhaltene Veresterungsgemisch gelangt dann in Kolonne K₁, in der der überschüssige Alkohol (ROH), Wasser und Leichtsieder (LS) über Kopf abdestilliert und das Estergemisch (EG) als Sumpf abgezogen und in die Fraktionierkolonne K₂ eingespeist wird. In dieser Kolonne wird das Gemisch in einer im wesentlichen von 1,4-Cyclohexandiolen freie Esterfraktion (EF) und eine Sumpffraktion, bestehend aus Hochsiedern (HS) und 1,4-Cyclohexandiolen (1,4-CHDO), fraktioniert. Die Esterfraktion (EF) wird dann in dem Hydrierreaktor R₂ katalytisch hydriert und das Hydriergemisch in der Destillationskolonne K₃ in Alkohol (ROH), Leichtsieder (LS) und reines 1,6-Hexandiol aufgetrennt.

### Variante B

Verwendet man zur Veresterung Alkohole mit 4 und mehr Kohlenstoffatomen, insbesondere n- oder i-Butanol, unterscheidet sich das Verfahren gemäß Fig. 2 nur insofern, als in der Fraktionskolonne K₂ das Estergemisch (EG) in ein Kopfprodukt von Niedrigsiedern (NS), die die 1,4-Cyclohexandiole (1,4-CHDO) enthalten, und eine im wesentlichen von 1,4-Cyclohexandiol freie Esterfraktion (EF) aufgetrennt wird, die man als Seitenfraktion oder als die Esterfraktion enthaltenden Sumpf gewinnt und in die Hydrierstufe (R₂) einspeist.

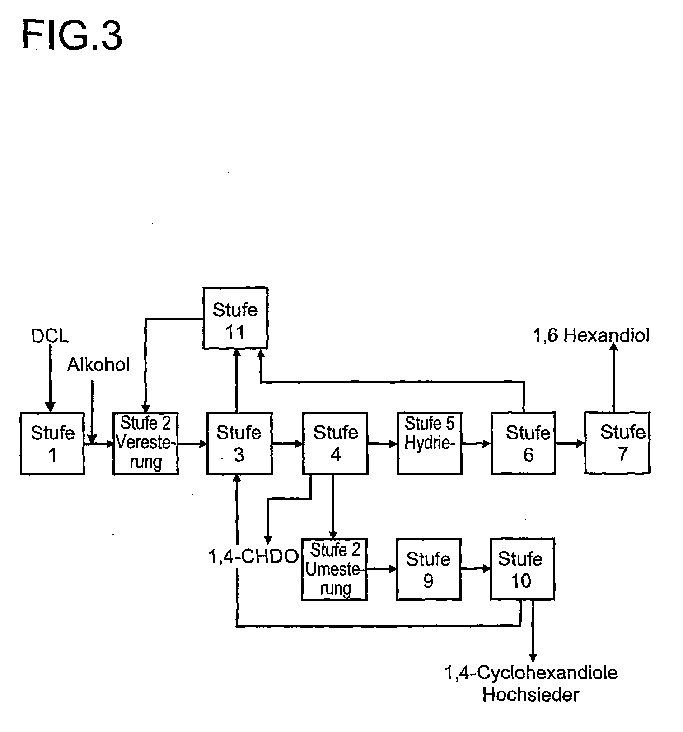

Im folgenden wird das Verfahren noch näher erläutert. Dabei sind gemäß Fig. 3 die einzelnen Verfahrensschritte in weitere Stufen aufgeschlüsselt, wobei die Stufen 2, 2a, 3, 4, 5, 6, 7 für das Verfahren essentiell sind und die Stufen 3 und 4 sowie 6 und 7 auch zusammengefasst werden können. Die Stufen 8, 9, 10 und 11 sind fakultativ, aber zur Erhöhung der Wirtschaftlichkeit des Verfahrens gegebenenfalls sinnvoll.

Die Dicarbonsäurelösung (DCL) ist im allgemeinen eine wässrige Lösung mit einem Wasseranteil von 20 bis 80 %. Da eine Vereiterungsreaktion eine Gleichgewichtsreaktion darstellt, ist es meist sinnvoll, insbesondere bei Veresterung mit z.B. Methanol, vorhandenes Wasser vor der Reaktion zu entfernen, vor allem, wenn während der Vereiterungsreaktion Wasser nicht, z.B. nicht azeotrop, entfernt werden kann. Die Entwässerung in Stufe 1 kann z.B. mit einem Membransystem erfolgen, oder bevorzugt durch eine Destillationsapparatur, bei der bei 10 bis 250°C, bevorzugt 20 bis 200°C, besonders bevorzugt 30 bis 200°C und einem Druck von 1 bis 1500 mbar, bevorzugt 5 bis 1100 mbar, besonders bevorzugt 20 bis 1000 mbar Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, dass das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.-% betragen.

Die Abtrennung des Wassers kann so erfolgen, dass das Wasser überwiegend säurefrei erhalten wird, oder man kann die in der DCL enthaltenen niederen Monocarbonsäuren - im wesentlichen Ameisensäure - zum größten Teil mit dem Wasser abdestillieren, damit diese in der Veresterung keinen Veresterungsalkohol binden.

Dem Carbonsäurestrom aus der Stufe 1 wird ein Alkohol mit 1 bis 10 C-Atomen zugemischt, gemäß Variante A Alkohole mit 1 bis 3 Kohlenstoffatomen, d.s. Methanol, Ethanol, Propanol oder iso-Propanol, bevorzugt Methanol, gemäß Variante B Alkohole mit 4 bis 10, insbesondere 4 bis 8 Kohlenstoffatomen und besonders bevorzugt n-Butanol, iso-Butanol, n-Pentanol und i-Pentanol.

Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor der Stufe 2, in dem die Carbonsäuren mit dem Alkohol verestert werden. Die Veresterungsreaktion kann bei 50 bis 400°C, bevorzugt bei 70 bis 300°C, besonders bevorzugt bei 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen; bevorzugt wird aber zur Erhöhung der Reaktionsgeschwindigkeit ein Katalysator zugesetzt. Dabei kann es sich um einen homogen gelösten oder um einen festen Katalysator handeln. Als homogene Katalysatoren seien beispielhaft Schwefelsäure, Phosphorsäure, Salzsäure, Sulfonsäuren, wie p-Toluolsulfonsäure, Heteropolysäuren wie Wolframatophosphorsäure oder Lewissäuren wie Aluminium-, Vanadium-, Titan-, Bor-Verbindungen genannt. Bevorzugt sind Mineralsäuren, insbesondere Schwefelsäure. Das Gew.-Verhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie SiO₂, Al₂O₃, SnO₂, ZrO₂ oder Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt, abzüglich der gegebenenfalls zugesetzten Säure als Katalysator, 0,01 bis 50, bevorzugt 0,1 bis 10. Dabei liegen nicht alle im System vorhandenen Carboxylgruppen als Ester des eingesetzten Alkohols vor, sondern ein Teil kann in Form von dimeren oder oligomeren Estern, z.B. mit dem OH-Ende der Hydroxycapronsäure vorliegen.

Das Veresterungsgemisch wird in Stufe 3, ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, - Carbonate, -Hydroxyde oder -Alkoholate, oder Amine in Substanz oder in dem Veresterungsalkohol gelöst verwendet.

Wird in Stufe 3 eine Kolonne verwendet, so erfolgt der Zulauf zur Kolonne bevorzugt zwischen dem Kopf- und dem Sumpfstrom. Über Kopf wird bei Drücken von 1 bis 1500 mbar, bevorzugt 20 bis 1000 mbar, besonders bevorzugt 40 bis 800 mbar und Temperaturen zwischen 0 und 150°C, bevorzugt 15 und 90°C, und insbesondere 25 und 75°C der überschüssige Veresterungsalkohol ROH, Wasser sowie z.B. entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren, wie Adipinsäure und Glutarsäure, Hydroxycarbonsäuren, wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 10 Gew.-% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 3 wird in die Stufe 4 eingespeist. Dabei handelt es sich um eine Destillationskolonne, bei der der Zulauf im allgemeinen zwischen den leichtsiedenden Komponenten und den schwersiedenden Komponenten erfolgt. Die Kolonne wird bei Temperaturen von 10 bis 300°C, bevorzugt 20 bis 270°C, besonders bevorzugt 30 bis 250°C und Drucken von 1 bis 1000 mbar, bevorzugt 5 bis 500 mbar, besonders bevorzugt 10 bis 200 mbar, betrieben.

Nach Variante A, d.h. der Veresterung mit C₁- bis C₃-Alkoholen, insbesondere Methanol, wird nun der Strom aus Stufe 3 in eine zu hydrierende Kopffraktion und eine die 1,4-Cyclohexandiole enthaltende Sumpffraktion getrennt.

Die Kopffraktion besteht überwiegend aus Restwasser und Restalkohol ROH, Estern des Alkohols ROH mit Monocarbonsäuren, überwiegend C₃- bis C₆-Monocarbonsäuren, Estern mit Hydroxycarbonsäuren, wie 6-Hydroxycapronsäure, 5-Hydroxyvaleriansäure, sowie vor allem den Diestern mit Dicarbonsäuren, wie Adipinsäure, Glutarsäure und Bernsteinsäure, ferner 1,2-Cyclohexandiolen, Caprolacton und Valerolacton.

Die genannten Komponenten können zusammen über Kopf abgetrennt und in die Hydrierung (Stufe 5) eingeschleust werden oder in einer weiteren bevorzugten Ausführungsform in der Kolonne in einen Kopfstrom, der überwiegend Restwasser und Restalkohol sowie die oben erwähnten Ester der C₃- bis C₅-Carbonsäuren enthält und einen Seitenstrom, der überwiegend die oben erwähnten Ester der C₆-Carbonsäuren und Dicarbonsäuren enthält, die dann in die Hydrierung gelangen, aufgetrennt werden.

Die schwersiedenden Komponenten des Stromes aus Stufe 4, überwiegend bestehend aus 1,4-Cyclohexandiolen oder deren Ester, dimeren oder oligomeren Estern sowie nicht näher definierten z.T. polymeren Bestandteilen der DCL, werden über den Abtriebsteil der Kolonne abgetrennt. Diese können zusammen anfallen oder so, dass über einen Seitenstrom der Kolonne im Abtriebsteil vorwiegend die 1,4-Cyclohexandiole und über Sumpf der Rest abgetrennt werden. Die so gewonnenen 1,4-Cyclohexandiole können z.B. als Ausgangsstoff für Wirkstoffe Verwendung finden. Die schwersiedenden Komponenten, mit oder ohne den Gehalt an 1,4-Cyclodiolen, können entweder verbrannt werden oder in einer bevorzugten Ausführungsform zur sog. Umesterung in die Stufe 8 gelangen.

Nach Variante B, d.h. der Veresterung mit C₄- bis C₁₀-Alkoholen, insbesondere n- oder i-Butanol, kann der Strom aus Stufe 3 in der Stufe 4 in eine die 1,4-Cyclohexandiole enthaltene Kopffraktion, einen vorwiegend die C₆-Ester enthaltenden Seitenstrom, der in die Hydrierung gelangt und Hochsieder enthaltenden Sumpfstrom, der gegebenenfalls in die Stufe 8 gelangen kann, aufgetrennt werden.

Die Kopffraktion besteht überwiegend aus Restalkohol ROH, C₁- bis C₃-Monoestern des Alkohols ROH, Valerolacton und 1,2- und 1,4-Cyclohexandiolen.

Der Seitenstrom enthält überwiegend Diester von Bernsteinsäure, Glutarsäure und Adipinsäure sowie Monoester der 5-Hydroxyvaleriansäure und 6-Hydroxycapronsäure. Dieser Seitenstrom kann entweder oberhalb oder auch unterhalb der Zulaufstelle der Kolonne entnommen werden und in die Hydrierung (Stufe 5) eingeschleust werden.

Der Sumpfstrom mit oligomeren Estern und sonstigen Hochsiedern kann analog der Variante A entweder verbrannt oder vorteilhaft in die Stufe 8 gelangen.

Nach einer weiteren Ausführungsform werden in der Stufe 4 die C₆-Ester zusammen mit entweder dem Sumpfstrom abgetrennt und dann, in einer weiteren Kolonne, entweder als Sumpfprodukt von der bereits beschriebenen Kopffraktion, die überwiegend aus Restalkohol ROH, C₁- bis C₃-Monoestern des Alkohols ROH, Valerolacton und 1,2- und 1,4-Cyclohexandiolen besteht, oder als Kopfstrom von den Hochsiedern abgetrennt.

Die von 1,4-Cyclohexandiole freie oder praktisch freie Fraktion der Stufe 4, entweder der Gesamtstrom oder der hauptsächlich Ester der C₆-Säuren enthaltende Seitenstrom, wird in die Hydrierstufe 5 geleitet.

Die Stufen 3 und 4 können, insbesondere wenn nur kleinere Mengen verarbeitet werden, zusammengefasst werden. Dazu kann beispielsweise in einer absatzweise durchgeführten fraktionierten Destillation der C₆-Esterstrom gewonnen werden, wiederum ohne dass 1,4-Cyclohexandiole in den zur Hydrierung geführten Strom gelangen.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind z.B. in Houben-Weyl, Methoden der Organischen Chemie, Band IV/1c, Georg Thieme Verlag Stuttgart, 1980, S. 45-67) und Beispiele für heterogene Katalysatoren sind z.B. in Houben-Weyl, Methoden der Organischen Chemie, Band IV/1c, S. 16 bis 26 beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. Cr₂O₃, Al₂O₃, SiO₂, ZrO₂, ZnO₂, BaO und MgO oder Mischungen daraus.

Besonders bevorzugt sind Katalysatoren, wie sie in EP 0 552 463 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c =̂ 0, d > 0, a > b/2, b > a/4, a > c und a > d gilt und x die zur Wahrung der Elektroneutralität pro Formeleinheit erforderliche Anzahl von Sauerstoffionen bezeichnet. Die Herstellung dieser Katalysatoren kann beispielsweise nach den Angaben der EP 552 463 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so dass man als besonders bevorzugte Fällungsmittel Alkalicarbonate oder Ammoniumcarbonat einsetzt. Wichtig für die Herstellung der Katalysatoren ist die thermische Behandlung der Zwischenstufen bei Temperaturen zwischen 500°C und 1000°C. Die BET-Oberfiäche der Katalysatoren liegt zwischen 10 und 150 m²/g.

Bevorzugt werden Heterogenkatalysatoren verwendet, die entweder fest angeordnet oder als Suspension eingesetzt werden. Wird die Hydrierung in der Gasphase und über fest angeordnetem Katalysator durchgeführt, werden im allgemeinen Temperaturen von 150 bis 300°C bei Drücken von 1 bis 100 bar, bevorzugt 15 bis 70 bar angewandt. Dabei wird zweckmäßig mindestens so viel Wasserstoff als Hydriermittel und Trägergas verwendet, dass Edukte, Zwischenprodukte und Produkte während der Reaktion nie flüssig werden. Der überschüssige Wasserstoff wird vorzugsweise im Kreis geführt, wobei ein kleiner Teil als Abgas zur Entfernung von Inerten wie z.B. Methan ausgeschleust werden kann. Es können dabei ein Reaktor oder mehrere Reaktoren hintereinander geschaltet verwendet werden.

Erfolgt die Hydrierung in der Flüssigphase mit fest angeordnetem oder suspendiertem Katalysator, so wird sie im allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinandergeschalteten Reaktoren durchgeführt werden. Die Hydrierung in Flüssigphase über ein Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden.

Die Hydrierung kann diskontinuierlich, bevorzugt kontinuierlich erfolgen.

Der Hydrieraustrag besteht im wesentlichen aus 1,6-Hexandiol und dem Alkohol ROH. Weitere Bestandteile sind, vor allem falls der gesamte leichtsiedende Strom der Stufe 4 gemäß Variante A eingesetzt wurde, 1,5-Pentandiol, 1,4-Butandiol, 1,2-Cyclohexandiole sowie kleine Mengen von Monoalkoholen mit 1 bis 6 C-Atomen und Wasser.

Dieser Hydrieraustrag wird in der Stufe 6, die z.B. ein Membransystem oder bevorzugt eine Destillationskolonne ist, in den Alkohol ROH, der zusätzlich den größten Teil der weiteren leichtsiedenden Komponenten enthält und einen Strom, der überwiegend 1,6-Hexandiol neben 1,5-Pentandiol und den 1,2-Cyclohexandiolen enthält, aufgetrennt. Dabei werden bei einem Druck von 10 bis 1500 mbar, bevorzugt 30 bis 1200 mbar, besonders bevorzugt 50 bis 1000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt. Der leichtsiedende Stoffstrom kann entweder direkt in die Veresterung der Stufe 2 zurückgeführt werden oder in die Stufe 8 oder in die Stufe 11 gelangen.

Der 1,6-Hexandiol enthaltene Stoffstrom wird in der Stufe 7 in einer Kolonne gereinigt. Dabei werden 1,5-Pentandiol, gegebenenfalls die 1,2-Cyclohexandiole, sowie weitere eventuell vorhandene Leichtsieder, über Kopf abgetrennt. Sollen die 1,2-Cyclohexandiole und/oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust. 1,6-Hexandiol wird mit einer Reinheit von mindestens 99 % aus einem Seitenstrom der Kolonne entnommen. Dabei werden bei Drücken von 1 bis 1000 mbar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Sollen nur kleinere Mengen 1,6-Hexandiol hergestellt werden, so können die Stufen 6 und 7 auch in einer diskontinuierlichen fraktionierten Destillation zusammengefasst werden.

Um die Hexandiol-Herstellung möglichst wirtschaftlich zu betreiben, ist es sinnvoll, den Veresterungsalkohol ROH zurückzugewinnen und immer wieder zur Veresterung einzusetzen. Dazu kann der vorwiegend den Alkohol ROH, beispielsweise Methanol enthaltende Strom aus Stufe 3 und/oder 6 in der Stufe 11 aufgearbeitet werden. Zu diesem Zweck wird erfindungsgemäß ein Membransystem verwendet. Membrantrennung kann in an sich bekannter Weise als Umkehrosmose, Pervarporation oder Dampfpermeation ausgeführt werden, wobei die Umkehrosmose bevorzugt ist. Die Grundprinzipien sowie die typischen Einsatzbedingungen dieser Membrantrennoperationen sind beispielsweise in T. Melin, R. Rautenbach, Membranverfahren - Grundlagen der Modul- und Anlagenauslegung, Springer-Verlag Berlin Heidelberg, 2.Auflage, 2004 oder in R. Baker, Membrane Technology and Applications, John Wiley & Sons , 2^{nd} Edition, 2004 beschrieben.

Bei der erfindungsgemäß bevorzugten Umkehrosmose strömt das zu trennende Gemisch unter einem Druck von 40 bis 300 bar an der Membran entlang. Das Permeat tritt durch die Membran hindurch. Die maximal erreichbare Konzentration im Retentat wird durch den osmotischen Druck der zurückgehaltenen Spezies bestimmt. Bei den verwendeten Membranen handelt es sich um Membranen mit porenfreie polymeren Trennschichten. Dabei kommen alle Membranpolymere in Betracht, die im Prozessmedium unter den angegebenen Trennbedingungen stabil sind. Die Membranen können in Flach-, Kissen- , Rohr-, Multikanalelement-, Kapillar- oder Wickelgeometrie ausgeführt werden, für die entsprechende Druckgehäuse, die eine Trennung zwischen Retentat und dem Permeat erlauben, verfügbar sind. Weiterhin können mehrere dieser Elemente in einem Gehäuse zu einem Modul zusammengefasst werden. Die Überströmgeschwindigkeit in dem Modul beträgt zwischen 0,05 und 8 m/s, besonders bevorzugt zwischen 0,1 und 4 m/s. Die transmembrane Druckdifferenz zwischen Permeat- und Retentatraum beträgt 20 bis 200 bar, im beanspruchten Verfahren bevorzugt zwischen 40 und 100 bar. Die Temperatur des Feedstroms, d.h. der vorwiegend den Alkohol ROH, beispielsweise Methanol enthaltende Strom aus Stufe 3 und/oder Stufe 6, zur Membranentrenneinheit beträgt zwischen 20 und 90°C.

Nach einer weiteren bevorzugten Ausführungsform des Verfahrens wird der hochsiedende Strom aus Stufe 4 (gemäß Variante A) zur Erhöhung der Gesamtausbeute an 1,6-Hexandiol bezogen auf eingesetzte Adipinsäure und 6-Hydroxycapronsäure in der eingesetzten DCL, verwendet. Dazu wird in der Stufe 8 der Anteil an dimeren und oligomeren Estern der Adipinsäure bzw. Hydroxycapronsäure mit weiteren Mengen des Alkohols ROH in Gegenwart eines Katalysators umgesetzt. Das Gew.-Verhältnis von Alkohol ROH und dem Sumpfstrom aus Stufe 4 beträgt zwischen 0,1 bis 20, bevorzugt 0,5 bis 10, besonders bevorzugt 1 bis 5. Als Katalysatoren eignen sich prinzipiell die bereits für die Veresterung in Stufe 2 beschriebenen. Bevorzugt werden jedoch Lewissäuren eingesetzt. Beispiele hierzu sind Verbindungen oder Komplexe des Aluminiums, Zinns, Antimons, Zirkons oder Titans, wie Zirkoniumacetylacetonat oder Tetraalkyltitanate, z.B. Tetraisopropyltitanat, die in Konzentrationen von 1 bis 10000 ppm, bevorzugt 50 bis 6000 ppm, besonders bevorzugt 100 bis 4000 ppm, bezogen auf das Umesterungsgemisch, angewandt werden. Besonders bevorzugt hierbei sind Titanverbindungen.

Die Umesterung kann absatzweise oder kontinuierlich, in einem Reaktor oder mehreren Reaktoren, in Reihe geschaltenen Rührkesseln oder Rohrreaktoren bei Temperaturen zwischen 100 und 300°C, bevorzugt 120 bis 270°C, besonders bevorzugt 140 bis 240°C und den sich dabei einstellenden Eigendrücken, durchgeführt werden. Die benötigten Verweilzeiten liegen bei 0,5 bis 10 Stunden, bevorzugt bei 1 bis 4 Stunden.

Dieser Strom aus der Stufe 8 lässt sich im Falle der Veresterung mit Methanol z.B. wieder in die Stufe 3 einschleusen. Zur Vermeidung von Aufpegelungen, vor allem von 1,4-Cyclohexandiolen, muss dann absatzweise oder kontinuierlich ein Teilstrom der Hochsieder aus Stufe 4 ausgeschleust werden. Eine andere Möglichkeit ist, den Strom aus Stufe 8 nicht in Stufe 3 zurückzuführen, sondern ihn, analog zur Stufe 3, in einer Stufe 9 in vorwiegend Alkohol ROH, der dann wieder in die Stufe 2, 8 oder 11 gelangen kann, und einen Strom, der die Ester enthält, aufzutrennen.

Dieser Esterstrom kann prinzipiell (mit der Maßgabe der Vermeidung von Aufpegelungen der 1,4-Cyclohexandiole) in die Stufe 4 zurückgeführt werden oder wird bevorzugt in eine weitere Stufe 10, in die Ester der C₆-Säuren und, mengenmäßig eher unbedeutend, in die Ester der C₅-Säuren einerseits, die entweder in die Stufe 4 oder direkt in die Stufe 5 eingeschleust werden können und Hochsieder andererseits, die die 1,4-Cyclohexandiole enthalten, aufgetrennt, worauf die Hochsieder ausgeschleust werden.

Auf diese Weise lassen sich Ausbeuten an 1,6-Hexandiol von über 95 %, bei Reinheiten von über 99 % erzielen.

Mit der erfindungsgemäßen Trennung der Alkohol-Leichtsieder-Gemische lässt das Verfahren gemäß WO 97/31883 wirtschaftlicher durchführen, da ein deutlich höherer Anteil des Vereesterungsalkohols in die Veresterung zurückgeführt werden kann.

Das Verfahren wird anhand des nachfolgenden Beispiels näher erläutert aber in keiner Weise eingeschränkt.

### Beispiel (Variante A)

### Stufe 1 (Entwässerung):

0,1 kg/h Dicarbonsäurelösung (bestehend im wesentlichen aus Adipinsäure, 6-Hydroxycapronsäure, 1,4-Cyclohexandiolen, Glutarsäure, 5-Hydroxyvaleriansäure, Ameisensäure, Wasser und mit einem Restkobaltgehalt von < 1 ppm) wurden kontinuierlich in eine Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden), mit aufgesetzter Füllkörperkolonne (ca. 4 theoretische Trennstufen, kein Rücklauf am Kopf) destilliert. Als Kopfprodukt wurden 0,045 kg/h erhalten mit einem Ameisensäuregehalt im Wasser von ca. 3 %. Im Sumpfstrom (5,5 kg) betrug der Wassergehalt ca. 0,4 %.

### Stufe 2 (Veresterung):

5,5 kg/h des Sumpfstroms aus Stufe 1 wurden mit 8,3 kg/h Methanol und 14 g/h Schwefelsäure kontinuierlich in einem Rohrreaktor (I 0,7 m, O 1,8 cm, Verweilzeit 2,7 h) umgesetzt. Die Säurezahl des Austrags abzüglich Schwefelsäure betrug ca. 10 mg KOH/g.

### Stufe 3 (Entfernen überschüssigen Alkohols und von Wasser):

In einer 20 cm Füllkörperkolonne wurde der Veresterungsstrom aus Stufe 2 destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 4 (Fraktionierung; 1,4-Cyclohexandiolabtrennung):

In einer 50 cm Füllkörperkolonne wurde der Sumpfstrom aus Stufe 3 fraktioniert destilliert (1 mbar, 70-90°C Kopftemperatur, bis 180°C Sumpftemperatur). Der Sumpf (1,9 kg) enthielt praktisch alle 1,4-Cyclohexandiole.

Als Leichtsieder wurden 0,6 kg abdestilliert (1,2-Cyclohexandiole, Valerolacton, 5-Hydroxyvaleriansäuremethylester, Glutarsäuredimethylester, Bernsteinsäuredimethylester u.a.). Als überwiegend Adipinsäuredimethylester und 6-Hydroxycapronsäuremethylester enthaltende Fraktion wurden 4,3 kg erhalten.

Der die Esterfraktion darstellende Kopfstrom wird in Hydrierstufe 5 geleitet.

### Stufe 5 (Hydrierung):

4,3 kg der C₆-Esterfraktion aus Stufe 4 wurden kontinuierlich in einem 25-ml-Reaktor an einem Katalysator hydriert (Katalysator, 70 Gew.-% CuO, 25 Gew.-% ZnO, 5 Gew.-% Al₂O₃), der zuvor im Wasserstoffstrom bei 180°C aktiviert worden war. Der Zulauf betrug 20 g/h, der Druck 220 bar und die Temperatur 220°C). Der Ester-Umsatz betrug 99,5 %, die 1,6-Hexandiolselektivität betrug über 99 %.

Alternativ wurde die Esterfraktion in einer zweistufigen Reaktorkaskade (1. Reaktor 2,5 I Katalysator, Rieselfahrweise, 250 bar, Produktrückführung : Zulauf = 10 : 1, 220 - 230°C; 2. Reaktor 0,5 I Katalysator, Rieselfahrweise gerader Durchgang, 260 bar, 220°C) kontinuierlich hydriert. Als Katalysator wurde ein zuvor bei 180°C aktivierter Katalysator aus CuO (60 %), Al₂O₃ (30 %) und Mn₂O₃ (10 %) eingesetzt. Die Zulaufmenge betrug 1 kg/h. Bei 99,5 % Umsatz betrug die Hexandiol-Selektivität über 99 %.

### Stufe 6 und 7 (Hexandiolreinigung):

4,0 kg des Hydrieraustrags aus Stufe 5 wurden fraktioniert destilliert (Destillationsblase mit aufgesetzter 70 cm Füllkörperkolonne, Rücklaufverhältnis 2) Bei 1013 mbar wurde 1 kg überweigend methanolisches Leichtsiedergemisch abdestilliert.
Nach Anlegen von Vakuum (20 mbar) destillierten überwiegend die 1,2-Cyclohexandiole und 1,5-Pentandiol ab. Danach (Sdp. 146°C) destillierte 1,6-Hexandiol mit einer Reinheit von 99,8 % ab. (Restgehalt überwiegend 1,5-Pentandiol.)

### Stufe 11

1 kg des Methanol-Leichsieder-Gemischs aus Stufe 6,7 und 7 kg/h aus Stufe 3 wurden gemischt zu einer Zusammensetzung von 80 Gew.-%, ca. 4,9-Gew.-% mTHF, etwa 1,5 Gew.-% THP und 12,1 Gew.-% mTHP. Von diesem Gemisch wurden 600 g in einer Rührdruckzelle, in die Flachmembranen mit einer freien Membranfläche von ca. 70 cm² eingebaut werden können, mit einer Umkehrosmosemembran des Typs Desal 3 SE der Firma GE Osminics, Minnetonka, USA bei einem durch Aufpressen von Stickstoff eingestellten Transmembrandruck von 80 bar um einen Massekonzentrationsfaktor (Masse eingesetztes Alkohol-Leichsieder-Gemisch :Masse Retentat) von etwa 3,6 bei einer Temperatur von 30°C aufkonzentriert. Das nach dem Membrandurchgang erhaltene Permeat und die zurückgehaltene Flüssigkeit (Retentat) wiesen folgende Zusammensetzung auf.

**Tabelle 1 :**

| | Permeat [Gew.-%] | Retentat [Gew.-%] |
|---|---|---|
| Methanol | ca. 88,9 | ca. 58,9 |
| MTHF | ca. 3,5 | ca. 8,5 |
| THP | ca. 0,9 | ca.3,0 |
| MTHP | ca. 6,1 | ca. 27,1 |
| Gewichtsanteil | 0,71 | 0,29 |

Es wurden 430 g des Permeats erhalten, das in Stufe 2 zurückgeführt wurde. Das Retentat wurde verworfen.

## Patentansprüche

1. Verfahren zur Herstellung von Hexandiol-1,6 aus einem Adipinsäure, 6-Hydroxycapronsäure und geringe Mengen 1,4-Cyclohexandiole enthaltenden Carbonsäuregemisch, das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemischs erhalten wird, durch Veresterung der Säuren mit C₁-bis C₁₀-Alkoholen und Hydrierung, wobei man
a) die in dem wässrigen Dicarbonsäuregemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag unter Abtrennung eines Alkohol-Leichtsieder-Gemischs in an sich bekannter Weise Hexandiol-1,6 gewinnt,
**dadurch gekennzeichnet, dass** aus den nach der Veresterung in Stufe b) und/oder nach der Hydrierung in Stufe e) gewonnenen Gemischen aus Alkoholen und Leichtsiedern durch ein Membransystem Alkohol abgetrennt und in die Veresterung zurückgeführt wird.

2. Verfahren nach Anspruch 1, **dadurch gekennzeichnet, dass** aus den in Stufe b) und Stufe e) gewonnenen Gemischen durch ein Membransystem Alkohol abgetrennt wird.

3. Verfahren nach Anspruch 1 oder 2, **dadurch gekennzeichnet, dass** man Methanol abtrennt.

4. Verfahren nach einem der Ansprüche 1 bis 3, **dadurch gekennzeichnet, dass** das Membransystem aus mindestens einer Membran besteht.

5. Verfahren nach einem der Ansprüche 1 bis 4, **dadurch gekennzeichnet, dass** die transmembrane Druckdifferenz 20 bis 200 bar beträgt.

6. Verfahren nach einem der Ansprüche 1 bis 4, **dadurch gekennzeichnet, dass** die Temperatur des in Stufe b) und/oder e) gewonnen Gemisches als Feedstrom der Membrantrennung 20 bis 90°C beträgt.

## Claims

1. A process for preparing 1,6-hexanediol from a carboxylic acid mixture which comprises adipic acid, 6-hydroxycaproic acid and small amounts of 1,4-cyclohexanediois and is obtained as a by-product in the oxidation of cyclohexane to cyclohexanone/cyclohexanol with oxygen or oxygen-containing gases and by water extraction of the reaction mixture, by esterification of the acids with C₁- to C₁₀-alcohols and hydrogenation, in which
a) the mono- and dicarboxylic acids present in the aqueous dicarboxylic acid mixture are reacted with a low molecular weight alcohol to give the corresponding carboxylic esters,
b) the resulting esterification mixture is freed of excess alcohol and low boilers in a first distillation stage,
c) a separation of the bottom product is carried out in a second distillation stage into an ester fraction substantially free of 1,4-cyclohexanediols and a fraction comprising at least the majority of the 1,4-cyclohexanediols,
d) the ester fraction substantially free of 1,4-cyclohexanediols is catalytically hydrogenated and
e) 1,6-hexanediol is obtained in a purifying distillation stage from the hydrogenation effluent while removing an alcohol-low boiler mixture in a manner known per se,
which comprises removing alcohol by a membrane system from the mixtures, obtained after the esterification in stage b) and/or after the hydrogenation in stage e), of alcohols and low boilers and recycling it into the esterification.

2. The process according to claim 1, wherein alcohol is removed by a membrane system from the mixtures obtained in stage b) and stage e).

3. The process according to claim 1 or 2, wherein methanol is removed.

4. The process according to any of claims 1 to 3, wherein the membrane system consists of at least one membrane.

5. The process according to any of claims 1 to 4, wherein the transmembrane pressure differential is from 20 to 200 bar.

6. The process according to any of claims 1 to 4, wherein the temperature of the mixture obtained in stage b) and/or e), as the feed stream of the membrane separation, is from 20 to 90°C.

## Revendications

1. Procédé de fabrication d'hexanediol-1,6 à partir d'un mélange d'acides carboxyliques contenant de l'acide adipique, de l'acide 6-hydroxycaproïque et de petites quantités de 1,4-cyclohexanediols, qui est obtenu en tant que produit secondaire lors de l'oxydation de cyclohexane en cyclohexanone/cyclohexanol avec de l'oxygène ou des gaz contenant de l'oxygène et par extraction aqueuse du mélange réactionnel, par estérification des acides avec des alcools en C₁ à C₁₀ et hydrogénation, selon lequel
a) les acides mono- et dicarboxyliques contenus dans le mélange aqueux d'acides dicarboxyliques sont mis en réaction avec un alcool inférieur pour former les esters d'acides carboxyliques correspondants,
b) le mélange d'estérification obtenu est débarrassé de l'alcool en excès et des composés de point d'ébullition bas lors d'une première étape de distillation,
c) une séparation en une fraction ester sensiblement exempte de 1,4-cyclohexanediols et une fraction contenant au moins la majeure partie des 1,4-cyclohexanediols est réalisée à partir du produit de fond lors d'une seconde étape de distillation,
d) la fraction ester sensiblement exempte de 1,4-cyclohexanediols est soumise à une hydrogénation catalytique et
e) l'hexanediol-1,6 est récupéré d'une manière connue en soi lors d'une étape de purification par distillation à partir de la sortie de l'hydrogénation, avec séparation d'un mélange alcool-composés de point d'ébullition bas,
**caractérisé en ce qu'**un alcool est séparé par un système membranaire à partir des mélanges d'alcools et de composés de point d'ébullition bas obtenus après l'estérification à l'étape b) et/ou après l'hydrogénation à l'étape e), et recyclé dans l'estérification.

2. Procédé selon la revendication 1, **caractérisé en ce qu'**un alcool est séparé par un système membranaire à partir des mélanges obtenus à l'étape b) et à l'étape e).

3. Procédé selon la revendication 1 ou 2, **caractérisé en ce que** du méthanol est séparé.

4. Procédé selon l'une quelconque des revendications 1 à 3, **caractérisé en ce que** le système membranaire est constitué d'au moins une membrane.

5. Procédé selon l'une quelconque des revendications 1 à 4, **caractérisé en ce que** la différence de pression transmembranaire est de 20 à 200 bar.

6. Procédé selon l'une quelconque des revendications 1 à 4, **caractérisé en ce que** la température du mélange obtenu à l'étape b) et/ou e) en tant que courant d'alimentation de la séparation membranaire est de 20 à 90 °C.
